# EUROPÄISCHE PATENTANMELDUNG

(11) **EP 1 709 930 A1**
(43) Veröffentlichungstag der Anmeldung: **11.10.2006**
(21) Anmeldenummer: 06007128.9
(22) Anmeldetag: 04.04.2006
(51) Int. Cl.: A61C 1/14, A61B 17/16, B23Q 1/70, B23B 31/20, B23B 31/26

(54) **Motorelement, insbesondere medizinisches Handstück mit einer Spannzange**

(30) Priorität: 07.04.2005 DE 102005016046; 12.04.2005 DE 102005016869
(71) Anmelder: Kaltenbach & Voigt GmbH, 88400 Biberach (DE)
(72) Erfinder: Düsing, Josef, 88299 Leutkirch (DE)
(74) Vertreter: Schmidt-Evers, Jürgen

(57) **Zusammenfassung**

Bei einem Motorelement, beispielsweise einem medizinischen oder dentaltechnischen Handstück oder einer Motorspindel, das wenigstens in seinem vorderen Bereich einen länglichen Schaft aufweist, in dem eine hohle Antriebswelle (3) drehbar gelagert ist, in deren vorderen Endbereich eine Spannzange (11) für ein Werkzeug angeordnet ist, die in ihrem hinteren Endbereich durch eine Kupplung mit einer sich nach hinten erstreckenden Stange verbunden ist, die durch die Kraft einer Feder nach hinten oder nach vorne vorgespannt ist und durch einen Betätigungsmechanismus gegen die Kraft der Feder nach vorne oder nach hinten verschiebbar ist, wobei die Feder nach hinten an der Zugstange abgestützt ist und nach vorne gegen eine innere erste Schulter der Antriebswelle abgestützt ist, ist am vorderen Endbereich der Feder (27) mindestens ein radial nach außen elastisch vorgespanntes Stützelement (25a) angeordnet ist, welches hinter die Schulter (52) ausfedert, wenn die Zugstange (16) mit der Feder von vorne in die Antriebswelle eingeschoben und das Stützelement (25a) in eine hinter der Schulter befindliche Position gelangt.

## Beschreibung

Die Erfindung betrifft insbesondere ein medizinisches Handstück nach dem Oberbegriff des Anspruchs 1.

Ein Handstück dieser Art ist in der DE 44 06 855 A1 beschrieben. Bei diesem vorbekannten Handstück ist eine Spannzange im vorderen Endbereich einer hohlen Antriebswelle angeordnet und rückseitig mit einer Zugstange zum Zusammendrücken der Spannzange verbunden, die zugleich ein Längsabschnitt der Antriebswelle ist. Die Zugstange ist durch eine zwischen ihr und der hohlen Antriebswelle angeordnete Druckfeder in Form einer Wendelfeder gebildet, die sich mit ihrem vorderen Ende an einer nach hinten gerichteten Schulter der hohlen Antriebswelle abstützt und mit ihrem hinteren Ende an einer nach vorne gerichteten Schulter der Zugstange angreift und somit die Zugstange nach hinten vorspannt. Dabei besteht die Antriebswelle im Bereich der Druckfeder aus zwei hülsenförmigen Teilen, die bezüglich den übrigen axialen Abschnitten der Antriebswelle in ihrer Querschnittsgröße erweitert sind und einander überlappen und fest miteinander verbunden sind. Bei dieser bekannten Ausgestaltung ergibt sich eine komplizierte Konstruktion, die nicht nur zu großen Querschnittsabmessungen führt, sondern auch eine aufwendige Montage bzw. Demontage vorgibt.

Der Erfindung liegt die Aufgabe zugrunde, ein medizinisches Handstück der eingangs angegebenen Art so auszugestalten, dass bei Gewährleistung einer einfachen Konstruktion die Montage bzw. Demontage mit einem geringen Aufwand erfolgen kann.

Diese Aufgabe wird durch die Merkmale des Anspruchs 1 gelöst. Vorteilhafte Weiterbildungen der Erfindung sind in den Unteransprüchen beschrieben.

Beim erfindungsgemäßen Handstück ist am vorderen Ende der Feder mindestens ein elastisch nach außen vorgespanntes Stützelement angeordnet, welches hinter die Schulter ausfedert, wenn die Stange mit der Feder von vorne in die Antriebswelle eingeschoben werden und das Stützelement in eine hinter der Schulter befindliche Position gelangt. Hierdurch ist nicht nur eine Konstruktion mit kleiner Querschnittsgröße erreichbar, sondern es ist auch eine Montage der Stange mit der Feder von vorne möglich, was zu einer wesentlichen Vereinfachung führt, weil das Handstück rückseitig für die Montage nicht geöffnet und danach wieder geschlossen werden muss. Die angestrebte Konstruktion kleinen Querschnitts ist deshalb möglich, weil bereits ein geringfügiges Hintergreifen der Schulter durch das Stützelement eine sichere und Abstützung der Feder nach vorne ermöglicht und deshalb das Handstück mit einer kleinen Querschnittsgröße realisierbar ist.

Das Stützelement lässt sich in einfacher und kostengünstiger Weise durch einen Federarm ausbilden, der aus einer entspannten Stellung, in der er die Schulter hintergreift, radial einwärts in eine Stellung einfederbar ist, in der er an die Innenquerschnittsgröße der Antriebswelle angepasst ist.

Die Einführung des Stützelements in die Antriebswelle lässt sich dadurch vereinfachen und handhabungsfreundlich gestalten, wenn rückseitig am Stützelement und/oder am vorderen Rand der Antriebswelle eine schräge oder gerundete Einführungsfläche angeordnet ist, die beim Einschieben der Stange mit der Feder das Stützelement selbsttätig in die Stellung einfedert, in der es in das Aufnahmeloch der Antriebswelle passt.

Die Schulter in der Antriebswelle kann durch eine Ringnut gebildet sein. Eine solche Ausgestaltung ist nicht nur einfach und kostengünstig herstellbar, sondern es braucht beim Einschieben der Stange mit der Feder auf keine besondere Drehpositionsstellung geachtet zu werden, weil das Stützelement in jeder Drehposition zur Schulterfläche passt.

Ein weiterer Vorteil der erfindungsgemäßen Ausgestaltung besteht darin, dass die Stange mit der Feder durch weiteres Einschieben von vorne nach hinten demontierbar ist, wozu ggf. das Handstück rückseitig zu öffnen, damit die Stange mit der Feder nach hinten ausgeschoben werden kann. Wenn dabei rückseitig am Stützelement oder am hinteren Rand der Ringnut schräge oder gerundete Einführungsflächen für das Stützelement vorgesehen sind, bewirken diese selbsttätig ein Einfedern des Stützelements.

Die erfindungsgemäße Ausgestaltung ermöglicht auch insofern eine einfache und kostengünstig herstellbare Konstruktion, weil das Aufnahmeloch in der Antriebswelle im wesentlichen über seine gesamte Länge hohlzylindrisch ausgebildet werden kann.

Im Rahmen der Erfindung können ein oder mehrere auf dem Umfang verteilt angeordnete Stützelemente vorgesehen sein. Dabei ist es vorteilhaft, dass oder die Stützelemente an einem Führungsring oder an einer Führungsbuchse auszubilden, in der die Zugstange gleitend geführt werden kann.

Weitere Weiterbildungen der Erfindung verbessern bei Gewährleistung von einfachen Ausgestaltungen das Vorschieben oder Zurückbewegen der Stange zum Zweck des Öffnens oder Schließens der Spannzange durch ein Druckglied, dass manuell von außerhalb des Handstücks zugänglich ist, und Rückführung durch Federkraft.

Im übrigen ist die vorliegende Erfindung nicht auf den medizinischen oder dentaltechnischen Einsatz beschränkt. Stattdessen könnte die erfindungsgemäße Lösung allgemein in motorgetriebenen Systemen bzw. Motorelementen mit einer Spanzannge für einen drehbares Werkzeug, beispielsweise in HF-Motorspindeln zum Einsatz kommen.

Nachfolgend werden vorteilhafte Ausgestaltungen der Erfindung anhand eines Ausführungsbeispiels und Zeichnungen näher erläutert. Es zeigt:
- Fig. 1: ein erfindungsgemäßes medizinisches Handstück im axialen Längsschnitt;
- Fig. 2: den vorderen Endbereich des Handstücks in nur geringfügig abgewandelter vergrößerter Darstellung;
- Fig. 3: eine Spannzange des Handstücks im axialen Schnitt;
- Fig. 4: die Spannzange in der Unteransicht;
- Fig. 5: die Spannzange in der Draufsicht;
- Fig. 6: eine Antriebswellen-Baueinheit im axialen Längsschnitt;
- Fig. 7: die in Fig. 6 mit X gekennzeichnete Einzelheit in vergrößerter Schnittdarstellung;
- Fig. 8: eine Zugstangen-Baueinheit im axialen Schnitt;
- Fig. 9: den vorderen Endbereich des Handstücks in weiter abgewandelter Ausgestaltung im axialen Längsschnitt;

- Fig. 10: ein Handstück in weiter abgewandelter Ausgestaltung im axialen Längsschnitt;
- Fig. 11: den vorderen Endbereich des Handstücks gemäß Fig. 10 in vergrößerter Darstellung;
- Fig. 12: einen Werkzeughalter in Form einer Spannzange des Handstücks gemäß Fig. 10 und 11 in vergrößerter Darstellung;
- Fig. 13: die Spannzange gemäß Fig. 12 in der Unteransicht.

Als bevorzugtes Ausführungsbeispiel für die vorliegende Erfindung wird nachfolgend ein medizinisches Handstück für ein medizinisches oder dentales Labor beschrieben, die erfindungsgemäße Konstruktion kann allerdings auch bei anderen Motorelementen Verwendung finden.

Das in seiner Gesamtheit mit 1 bezeichnete Handstück eignet sich aufgrund seiner stabilen und hinreichend großen Konstruktion zur spanabhebenden Bearbeitung von Modellen, künstlichen Teilen wie Prothesen oder Abdrücken des menschlichen oder tierischen Körpers. In der Fachsprache wird ein solches Handstück 1 mit medizintechnisches oder dentaltechnisches Handstück oder Arbeitshandstück bezeichnet. Für eine Zahnbehandlung im Mundraum eines Patienten ist das Ausführungsbeispiel wegen seiner Konstruktionsgröße weniger gut geeignet. Jedoch eignet sich die beim Ausführungsbeispiel realisierte Erfindung prinzipiell auch für solche Handstücke, die sich für die Zahnbehandlung im Mundraum eines Patienten eignen. Gleiches gilt auch für den medizinischen Bereich.

Die Hauptteile des Handstücks 1 sind ein dem manuellen Ergreifen dienender Schaft 2, der sich wenigstens in seinem vorderen Bereich gerade erstreckt und beim Ausführungsbeispiel sich insgesamt gerade erstreckt, eine sich im Schaft 2 längs erstreckende Antriebswelle 3, die in einem vorderen und einem hinteren Drehlager 4, 5 drehbar und axial unverschiebbar im Schaft 2 gelagert ist, ein vorzugsweise elektrischer Antriebsmotor 6, der im Schaft 2 als Drehantrieb für die Antriebswelle 3 angeordnet ist und eine Spannvorrichtung 7 für ein andeutungsweise dargestelltes Werkzeug 8, das mit einem Werkzeugschaft 8a in ein Steckloch 9 der Spannvorrichtung 7 einsteckbar und spannbar sowie wieder lösbar ist.

Die Spannvorrichtung 7 weist einen Werkzeughalter 12 auf, der vorzugsweise durch eine sogenannte Spannzange 11 gebildet ist, die beim Ausführungsbeispiel in der Antriebswelle 3 in ihrer Längsrichtung verschiebbar gelagert ist und mehrere, z.B. drei, auf dem Umfang verteilt und endseitig angeordnete Spannsegmente 11a aufweist, die durch einen Spannmechanismus gegen das Werkzeug 8 bzw. den Werkzeugschaft 8a spannbar sind sowie durch einen von außen manuell zugänglichen Lösemechanismus 13 wieder lösbar sind. Für den Spann- und Lösevorgang ist die Spannzange 11 zwischen einer Spannstellung und einer Lösestellung längs verschiebbar. Beim Ausführungsbeispiel werden die Spannsegmente 11a vorzugsweise beim Zurückbewegen der Spannzange 11 radial einwärts gegen das Werkzeug 8 bzw. dessen Schaft 8a zusammengedrückt. Hierzu dient ein Spannkonus 15 mit einem Innenkonus 15a in der Antriebswelle 3 und dazu passenden Außenkonusflächen 15b an den Spannsegmenten 11a. In der nach vorne verschobenen Lösestellung der Spannzange 11 sind die Spannsegmente 11a ohne Spanndruck, so dass der Werkzeugschaft 8a in die Spannzange 11 eingesteckt bzw. herausgezogen werden kann. Damit bei fehlendem Werkzeug 8 die Spannsegmente 11a nicht zu weit radial nach innen bewegt werden, sind an den vorderen Enden der Spannsegmente 11a Flanschstücke 11b angeordnet, die die Bewegung der Spannzange 8 axial nach innen durch Anlage an der Antriebswelle 3 begrenzen.

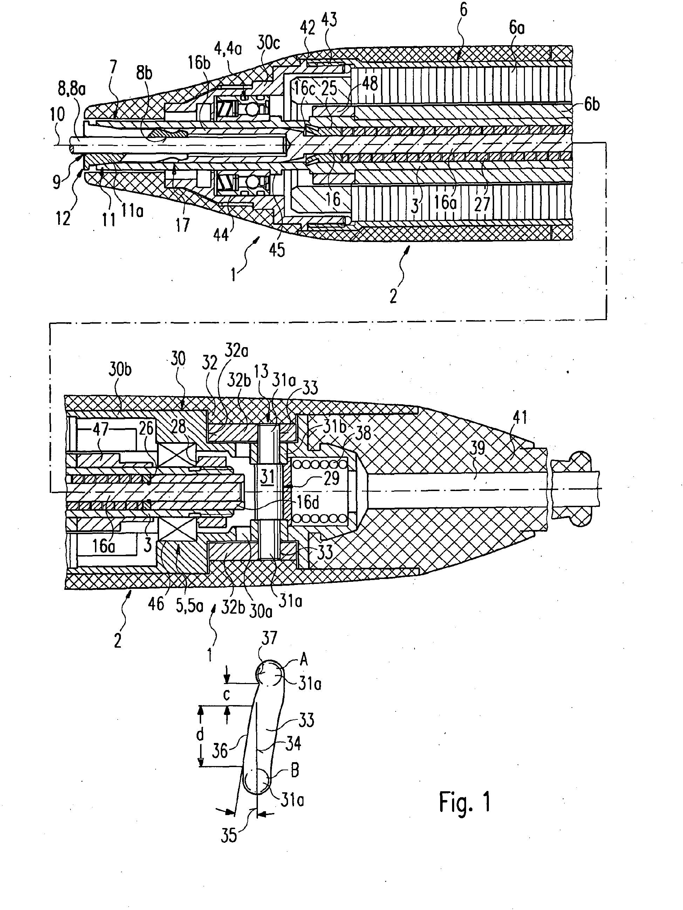

Zum längs gerichteten Hin- und Herbewegen der Spannzange 11 dient eine sich längs erstreckende Stange 16, beim Ausführungsbeispiel gemäß Fig. 1 eine Zugstange , die koaxial in der Antriebswelle 3 verschiebbar gelagert ist und an ihrem vorderen Ende lösbar mit dem hinteren Ende der Spannzange 11 verbunden ist. Hierzu ist zwischen der Stange 16 und der Spannzange 11 eine axial und formschlüssig wirksame Kupplung 17 mit einem ersten Kupplungselement 17a an dem einen zu kuppelnden Teil und einem zweiten Kupplungselement 17b an dem anderen zu kuppelnden Teil angeordnet, wobei das erste Kupplungselement 17a zwischen einer das andere Kupplungselement 17b in einer Hinterschneidung 18 formschlüssig hintergreifenden Kupplungsstellung und einer aus der Hinterschneidung 18 heraus bewegten Freigabestellung bewegbar und durch ein Arretierelement 8b in der Kupplungsstellung arretierbar ist. Durch die Arretierung ist gewährleistet, dass das bewegbare Kupplungselement 17a das andere Kupplungselement 17b in der Kupplungsstellung nicht nur formschlüssig hintergreift sondern auch darin gegen ein Herausbewegen arretiert ist. Hierdurch ist die Kupplungssicherheit gewährleistet, so dass die Kupplung 17 axiale Kräfte von der Stange 16 auf die Spannzange 11 oder umgekehrt übertragen kann.

Die Erfindung ist nicht auf einen Werkzeughalter 12 in der Form einer Spannzange 11 beschränkt. Eine Spannvorrichtung 7 kann im Rahmen der Erfindung z.B. im vorderen Endbereich des Werkzeughalters 12 angeordnet sein und z.B. eine Klemmschraube aufweisen, mit der ein Werkzeug am Werkzeughalter festklemmbar ist. Deshalb wird im folgenden die Beschreibung mit dem Bauteil Werkzeughalter 12 fortgesetzt, sofern eine Spannzange 11 nicht erforderlich ist.

Im Rahmen der Erfindung kann das radial bewegbare erste Kupplungselement 17a an der Stange 16 und das radial starre zweite Kupplungselement 17b am Werkzeughalter 12 angeordnet sein. Beim Ausführungsbeispiel ist die umgekehrte Anordnung vorgesehen. Das erste Kupplungselement 17a ist am Werkzeughalter 12 und das zweite Kupplungselement 17b ist an der Stange 16 angeordnet. Ein solcher Werkzeughalter 12 weist einen hinteren Werkzeughalterabschnitt auf, mit dem er in die im vorderen Endbereich hohl ausgebildete Antriebswelle 3 bis in den Bereich der Kupplung 17 einsteckbar ist. Zur Bewegung des ersten Kupplungselements 17a in die Hinterschneidung 17c erfolgt vorzugsweise selbsttätig durch ein das erste Kupplungselement 17a in seine hintergreifende Kupplungsstellung vorspannenden Federelement. Beim Ausführungsbeispiel ist das erste Kupplungselement 17a an einem vom Werkzeughalterkörper nach hinten erstreckenden Federarm 19 angeordnet, von dem es radial nach außen absteht. Das erste Kupplungselement 17a und der Federarm 19 sind durch radiale Längsschlitze 21 vom übrigen Körper des Werkzeughalters 12 getrennt, die am zugehörigen Ende axial ausmünden.

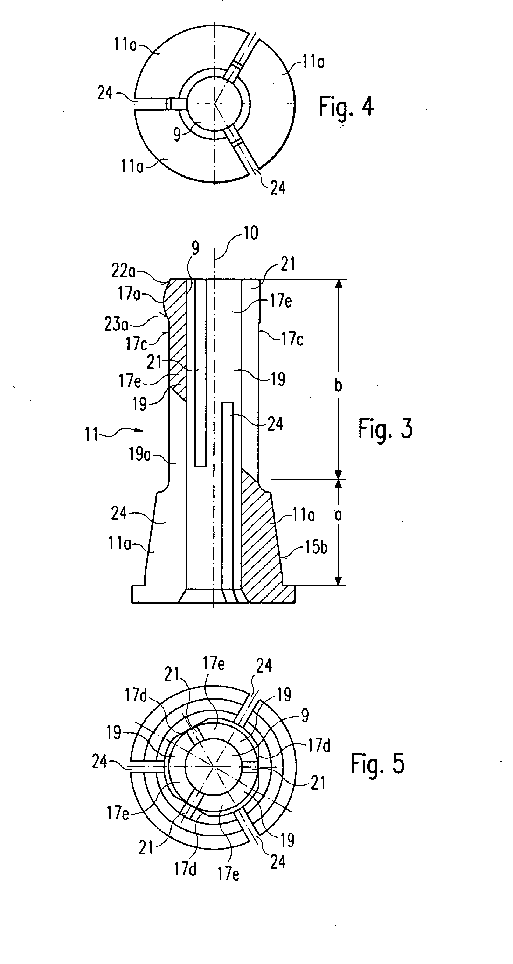

Es ist zur Erzielung einer Drehsicherung vorteilhaft, die beim Ausführungsbeispiel hinter dem zweiten Kupplungselement 17b, nämlich dem Gegenkupplungselement, vorhandene Hinterschneidung 17c mit das erste Kupplungselement 17a in beiden Umfangsrichtungen mit Bewegungsspiel begrenzenden Ausnehmungsflächen 17d (Fig. 5) auszubilden. Hierzu ergibt sich eine in beide Umfangsrichtungen formschlüssig wirksame Drehsicherung für den Werkzeughalter 12 in der Antriebswelle 3. Es ist aber auch vorteilhaft, die Hinterschneidung 17c als Ringnut bzw. als sich in den Spannsegmenten 11a in der Umfangsrichtung fortsetzende Nut auszubilden. In einem solchen Fall ist zwar keine Drehsicherung im Bereich der Kupplung 17 vorhanden, jedoch wird hierdurch der Vorteil erreicht, dass der Werkzeughalter 12 in jeder wahlweisen Drehstellung in die Kupplung 17 eingeschoben und gekuppelt werden kann. Eine Drehsicherung kann dabei auf eine andere Art und Weise realisiert sein. Im Falle der Ausbildung des Werkzeughalters 12 als Spannzange 11 ist eine Drehsicherung durch den Klemmkonus der Spannzange 11 geschaffen.

Um die Montage und/oder Demontage des Werkzeughalters 12 zu erleichtern, ist es vorteilhaft, dass erste Kupplungselement 17a und/oder das zweite Kupplungselement 17b mit vorderen bzw. hinteren schrägen oder gerundeten Einführungsflächen 22a, 22b bzw. Ausführungsflächen 23a, 23b auszubilden, die bezüglich der Längsmittelachse und Drehachse 10 der Antriebswelle 3 eine so große Neigung aufweisen, dass der Werkzeughalter 12 mit einer handhabungsfreundlich aufbringbaren Axialkraft in die Kupplung 17 eingeschoben und/oder aus der Kupplung 17 herausgezogen werden kann, wobei das bewegbare Kupplungselement 17a selbsttätig ausfedert. Hierdurch ist eine Verrastungsvorrichtung gebildet, deren Verrastungskräfte so groß sind, dass im Funktionsbetrieb die axiale Kupplung des Werkzeughalters 12 gewährleistet ist und für eine Montage und/oder Demontage des Werkzeughalters 12 die Kupplung 17 mit einem axialen manuell aufbringbaren Kraftaufwand zu überdrücken ist.

Beim Ausführungsbeispiel, bei dem mehrere, vorzugsweise drei, bewegbare erste Kupplungselemente 17a mit zugehörigen zweiten Kupplungselementen 17b mit Hinterschneidungen 17c auf dem Umfang verteilt angeordnet sind, sind die bewegbaren Kupplungselemente 17a mit den zugehörigen Federarmen 19 jeweils als einander gleiche endseitig angeordnete Segmente ausgebildet, die durch vorzugsweise drei endseitig ausmündende Längsschlitze 21 voneinander getrennt sind. Die in die Umfangsrichtung gerichtete Breite der Längsschlitze 21 ist so groß, dass die bewegbaren Kupplungselemente 17a jeweils ausfedern können, wobei die vorhandene Breite der Längsschlitze 21 sich jeweils etwas verringert.

Wenn der Werkzeughalter 12 als eine Spannzange 11 mit mehreren, vorzugsweise drei, auf dem Umfang verteilt angeordneten Spannsegmenten 11a ausgebildet ist, die durch Längsschlitze 24 voneinander getrennt und an dadurch gebildeten Federarmen 19a angeordnet sind, ist es zur Verringerung der Baulänge vorteilhaft, die Längsschlitze 21 bezüglich den Längsschlitzen 24 in die Umfangsrichtung versetzt zueinander anzuordnen, wobei die Längsschlitze 21, 24 einander überlappen können, wie es Fig. 3 deutlich zeigt.

Der hülsenförmige Werkzeughalter 12 weist ein hohlzylindrisches Steckloch 9 auf, einen vorderen konischen Längsabschnitt a und einen sich von diesem nach hinten erstreckenden im wesentlichen hohlzylindrischen Längsabschnitt b, die jeweils durch die vorbeschriebenen Segmente gebildet sind. Die Querschnittsform und -größe des Stecklochs 9 ist mit geringem Bewegungsspiel an die Querschnittsform und -größe des Werkzeugs 8 bzw. Werkzeugschaftes 8a angepasst. Die Wandabschnitte des vorzugsweise hohlzylindrischen Stecklochs 9 bilden somit im Bereich der Spannsegmente 11a hohlzylinderabschnittförmige Spannflächen und im Bereich der Kupplungssegmente hohlzylinderabschnittförmige Anschlagflächen, mit denen die Kupplungssegmente 7e an der Umfangsfläche des Werkzeugs 8 bzw. Schaftes 8a anliegen.

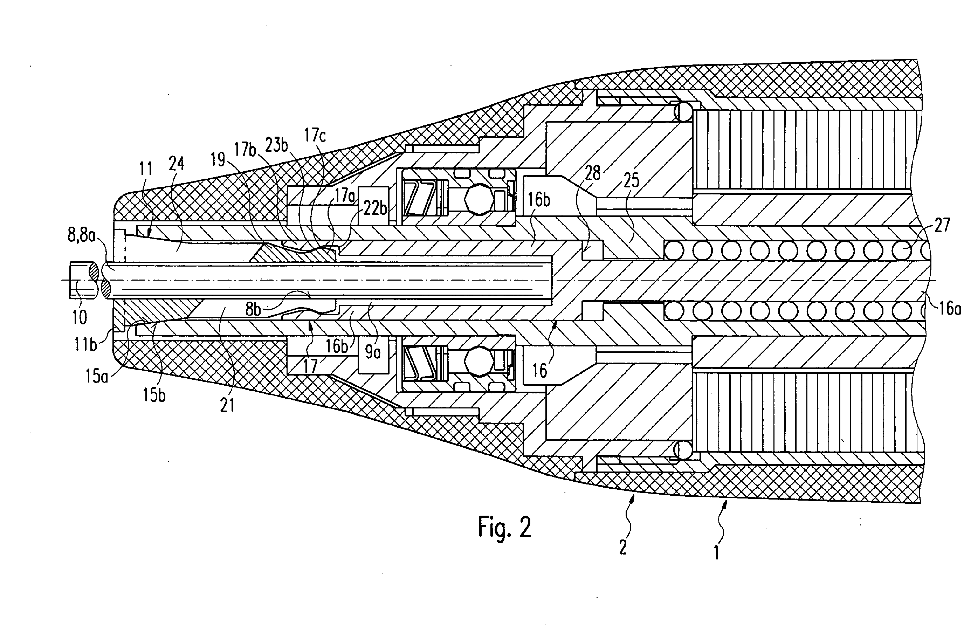

Die Ausgestaltungen gemäß Fig. 1 bzw. 2 zum einen und Fig. 5 zum anderen unterscheiden sich durch die Mantelflächenform der Kupplungselemente 17e. Gemäß Fig. 1 bzw. 2 sind die Kupplungselemente 17e vor der zugehörigen Hinterschneidung 17c verdickt, wobei die Hinterschneidungen 17c im Längsquerschnitt konkav gerundet ist. Gemäß Fig. 5 erstreckt sich die Hinterschneidung 17c vor den konvexen Kupplungselementen 17a im wesentlichen zylindrisch nach vorne.

Die Stange 16 erstreckt sich beim Ausführungsbeispiel von der Kupplung 17 nach hinten längs durch die hülsenförmige Antriebswelle 3 hindurch, wobei sie vorzugsweise das hintere Ende der Antriebswelle 3 überragt. Hinter dem vorderen Endbereich der Stange 16, in dem ein Steckloch 9a angeordnet ist, erstreckt sich die Stange 16 mit einem im Querschnitt verjüngten Stangenabschnitt 16a, der in seinem vorderen Endbereich in einer Führungsbuchse 25 in der Antriebswelle 3 längs verschiebbar geführt ist. Zwischen dem hinteren Ende der Führungsbuchse 25 und einer nach vorne gerichteten Schulterfläche 26 im hinteren Endbereich der Stange 16 befindet sich eine Druckfeder 27 in Form einer Wendelfeder auf dem verjüngten Stangenabschnitt 16a, die die Stange 16 nach hinten vorspannt. Bei demontiertem Werkzeughalter 11 liegt der verdickte vordere Endbereich 16b der Stange 16 mit einer rückseitigen Schulterfläche 16c an der Führungsbuchse 25 an. Die davon rückseitig angeordnete Schulterfläche 26 kann durch eine auf den Stangenabschnitt 16a aufgeschobene und darauf fixierte Buchse 16d gebildet sein, mit der der hintere Endbereich der Stange 16 in der hülsenförmigen Antriebswelle 3 axial verschiebbar gelagert ist. Die Führungsbuchse 25 in Fig. 2 weicht von den übrigen Ausführungsbeispielen insofern ab, als sie durch einen inneren Ringansatz der Antriebswelle 3 gebildet ist.

In der Normalstellung ist die durch die Feder 27 nach hinten vorgespannte Stange 16 in ihrer nach hinten gerichteten Bewegung dadurch begrenzt, dass bei einem in die Spannzange 11 eingesteckten Werkzeug 8 der Außenkonus der Spannzange 11 am Innenkonus der Antriebswelle 3 anliegt. Zum Lösen des Werkzeugs 8 wird die Stange 16 durch den Betätigungsmechanismus 13 nach vorne bewegt. Dies kann durch ein Druckglied 29 erfolgen, das z. B. als vorzugsweise zylindrischer Querstift 31 ausgebildet ist, der durch den Betätigungsmechanismus 13 nach vorne gegen das hintere Ende der Stange 16 (Teil 16a und/oder 16d) bewegbar ist. Beim Ausführungsbeispiel ist der Querstift 31 im Bereich seiner z. B. verjüngten Enden 31a in Nuten 33 verschiebbar gelagert, die sich mit einer Steigung in der Umfangsrichtung des Handstücks 1 in einer Betätigungshülse 32 befinden, die in einer Ringnut 32a des Handstücks 1 axial unverschiebbar und in Umfangsrichtung drehbar gelagert ist. Eine der beiden Nuten 33 ist in Fig. 1 als Abwicklung beispielhaft dargestellt. Die vorzugsweise in einer Innenbuchse 32b, insbesondere aus verschleißfestem Material, z.B. Stahl, angeordneten Nuten 33 erstrecken sich in der Umfangsrichtung derart schräg oder gerundet, dass ausgehend von der in Fig. 1 dargestellten Spannstellung bei einer Relativverdrehung der Betätigungshülse 32 das Druckglied 29 nach vorne verdrängt wird und dabei die Stange 16 nach vorne bewegt und die Spannzange 11 löst. In dieser Lösestellung befinden sich die Enden 31a des Druckglieds 29 in den Endbereichen B der Nuten 33. Dabei kann die Anordnung so getroffen sein, dass der der Lösestellung zugehörige Endbereich B der Nuten 33 nicht schräg sondern in die Umfangsrichtung gerichtet ist, so dass ein selbsttätiges Rückdrehen der Betätigungshülse 32 aufgrund der Federspannung und ggf. aufgrund der Wirkung von Vibrationen, verhindert ist. Die bezüglich einer zugehörigen, rechtwinklig zur Längsmittelachse gerichteten Querebene 35 schräg nach vorne verlaufende Neigung 36 kann im Bereich der Länge der Nuten 33 unterschiedlich sein. Beim Ausführungsbeispiel ist ausgehend vom der Spannstellung zugehörigen Endbereich A ein verhältnismäßig stark geneigter Neigungsverlauf c vorgesehen, an den sich ein weniger stark geneigter Neigungsverlauf d anschließt, an den sich ein in die Umfangsrichtung gerichteter Endbereich B der Nuten 33 anschließt. In den Endbereichen B der Nuten 33, in denen sich die Druckgliedenden 31a in einer der Spannstellung entsprechenden Stellung befinden, sind vorzugsweise Rastvertiefungen 37 vorgesehen, in denen die Enden 31a einrasten können, und die beim Ausführungsbeispiel nach vorne gerichtet sind. Durch eine hinter dem Druckglied 29 angeordnete Feder 38, bspw. eine Wendel-Druckfeder, lässt sich das Druckglied 29 nach vorne vorspannen und somit auch in die Rastvertiefungen 37 vorspannen. Beim Drehen der Betätigungshülse 32 in die Lösestellung B werden das Druckglied 29, die Stange 16 und die Spannzange 11 in die Lösestellung vorbewegt. Beim Zurückdrehen der Betätigungshülse 32 in die Spannstellung A wird das Druckglied 29 wieder in die beabstandete Spannstellung (Freistellung) zurückbewegt, in der es die Stange 16 freigibt und die Spannzange 11 aufgrund der vorhandenen axialen Federkraft gespannt wird.

Bei den vorbeschriebenen Vor- und Rückbewegungen des Druckglieds 29 wird es z. B. mittels auf ihm sitzenden Gleithülsen 31b in Längsnuten 30a einer tragenden Umfangswand 30b eines Handstück-Gehäuses 30 längs geführt.

Als Drehantrieb für die Antriebswelle 3 ist der Elektromotor 6 vorgesehen, der im Handstück 1 angeordnet ist und z.B. mit einem hülsenförmigen Stator 6a innen an der Umfangswand des vorhandenen Handstückgehäuses angeordnet ist und mit seinem hülsenförmigen Rotor 6b auf der Antriebswelle 3 sitzt. Eine andeutungsweise dargestellte elektrische Stromleitung 39 erstreckt sich von hinten durch eine flexible Kabeldurchführung 41 aus elastisch verformbarem Material, die mit einem hinteren Gehäuseabschnitt des Handstückgehäuses verbunden ist.

Das Gehäuse 30 besteht aus zwei tragenden Umfangswänden, nämlich der hinteren längeren Umfangswand 30b und einer vorderen kürzeren Umfangswand 30c, die im vorderen Bereich des Gehäuses 30 einander überlappen und in diesem Bereich durch ein Außengewinde und ein darin einfassendes Innengewinde miteinander verschraubt sind und an einer Teilungsfuge 42 aneinanderliegen. Die z. B. hinter der Teilungsfuge 42 angeordnete Verschraubung ist mit 43 bezeichnet. Die hintere Umfangswand 30b ist im wesentlichen hohlzylindrisch ausgebildet und in ihrem Bereich ist der Stator 6a angeordnet. Der vordere Handstückabschnitt und die vordere Umfangswand 30c verjüngen sich zum vorderen Ende des Handstücks 1 hin.

Das vordere Drehlager 4 ist in der vorderen Umfangswand 30c angeordnet und durch ein Wälzlager 4a gebildet, das außen in einer nach hinten offenen Lagerbohrung 44 in der vorderen Umfangswand 30c sitzt und innen auf der hohlen Antriebswelle 3 sitzt, wobei es rückseitig von einem Ringbund 45 auf der Antriebswelle 3 begrenzt ist.

Das hintere Drehlager 5 ist ebenfalls durch ein Wälzlager gebildet, das außen in einer nach vorne offenen Lagerbohrung 46 im hinteren Endbereich der hinteren Umfangswand 30b sitzt und innen auf der Antriebswelle 3 sitzt. Der Innenring des hinteren Wälzlagers 5a ist an seiner Vorderseite durch den Rotor 6b oder eine darin anliegende Zwischenhülse 47 begrenzt. Der Rotor 6b ist an seinem vorderen Ende durch einen Ringbund 48 auf der Antriebswelle 3 begrenzt, der auch von dem Ringbund 45 gebildet sein könnte. Rückseitig vom hinteren Wälzlager 5a befindet sich eine Gewindemutter 49, die von hinten auf einen Gewindeabschnitt der Antriebswelle 3 aufgeschraubt ist und den Innenring sowie die weiteren vorderseitig von diesen auf der Antriebswelle 3 angeordneten Ringteilen gegen die Ringbund 48 drückt und axial festliegt.

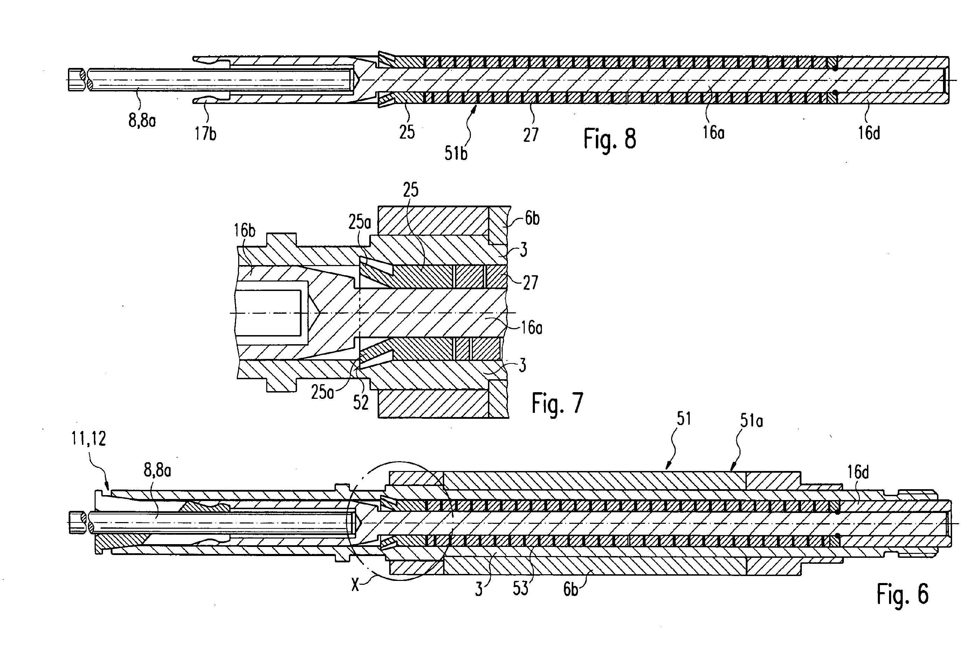

Dabei bilden die Antriebswelle 3 mit dem Rotor 6b und der Stange 16 mit der Druckfeder 27 eine vormontierbare Baueinheit 51, siehe Fig. 6.

Beim vorliegenden Ausführungsbeispiel besteht diese Baueinheit 51 aus zwei Baueinheiten 51a, 51b, wobei die Baueinheit 51a die Antriebswelle 3, den Rotor 6b, umfaßt, während die zweite Baueinheit 51b die Stange 16, die Buchse 16d, die Druckfeder 27 und die Führungsbuchse 25 umfaßt.

Zur axialen Positionierung dieser Baueinheit 51b ist in der Antriebswelle 3 hinter dem verdickten Endbereich 16b eine oder mehrere nach hinten gerichtete Schulterflächen 52 vorgesehen, gegen die die Führungsbuchse 25 in Richtung nach vorne begrenzt und positioniert ist. Die Druckfeder 27 ist rückseitig an der Buchse 16d abgestützt, und sie spannt die Führungsbuchse 25 nach vorne vor, wobei sie aufgrund der axialen Abstützung der Führungsbuchse 25 an der wenigstens einen Schulterfläche 52 die Stange 16 nach hinten vorspannt. Im montierten Zustand ist dabei die Stange 16 oder die Buchse 16d rückseitig am Druckglied 29 abgestützt. Diese Abstützung kann aber auch dadurch erfolgen, daß die Spannzange die Spannsegmente 11a oder die Flanschstücke 11b rückseitig durch die Antriebswelle 3 begrenzt sind.

Beim vorliegenden Ausführungsbeispiel ist die innere Baueinheit 51b von vorne in die hohle Antriebswelle 3 einsteckbar und montierbar. Dies wird dadurch erreicht, daß die Führungsbuchse 25 an ihrem vorderen Ende einen oder mehrere auf dem Umfang verteilt angeordnete Federarme 25a aufweist, deren vordere Enden im entspannten Zustand bezüglich der Längsmittelachse 10 ein Radialmaß einnehmen, das größer ist, als das Radialmaß des vorzugsweise auf seiner gesamten Länge zylindrischen Aufnahmelochs 53 in der Antriebswelle. Die Schulterfläche 52 ist durch eine Ringnut im Aufnahmeloch 53 gebildet.

Zur Montage der inneren Baueinheit 51b in die Antriebswelle 3 wird die Baueinheit 51b von vorne in das Aufnahmeloch 53 eingeschoben, bis der oder die dabei einfedernden Federarme 25a hinter der wenigstens einen Schulterfläche 52 ausfedern und die Baueinheit 51a nach vorne an der wenigstens einen Schulterfläche 52 positionieren.

Im demontierten Zustand liegt aufgrund der Federspannung der Druckfeder 27 die Führungsbuchse 25 an der ihr vorgeordneten Schulterfläche 16c an. Im montierten Zustand ist dagegen zwischen der Schulterfläche 16c und der Führungsbuchse 25 ein axiales Bewegungsspiel auch im gespannten, d. h. nach hinten verschobenen Zustand der Spannsegmente 11a vorhanden, damit die radial einwärts gerichtete Spannwirkung der Spannsegmente11a nicht beeinträchtigt wird.

Für eine Demontage der Baueinheit 51b wird nach einer Entfernung des Werkzeughalters 9 bzw. der Spannsegmente 11a die Stange 16 nach hinten herausgeschoben, wobei die Federarme 25a unbeschädigt einfedern.

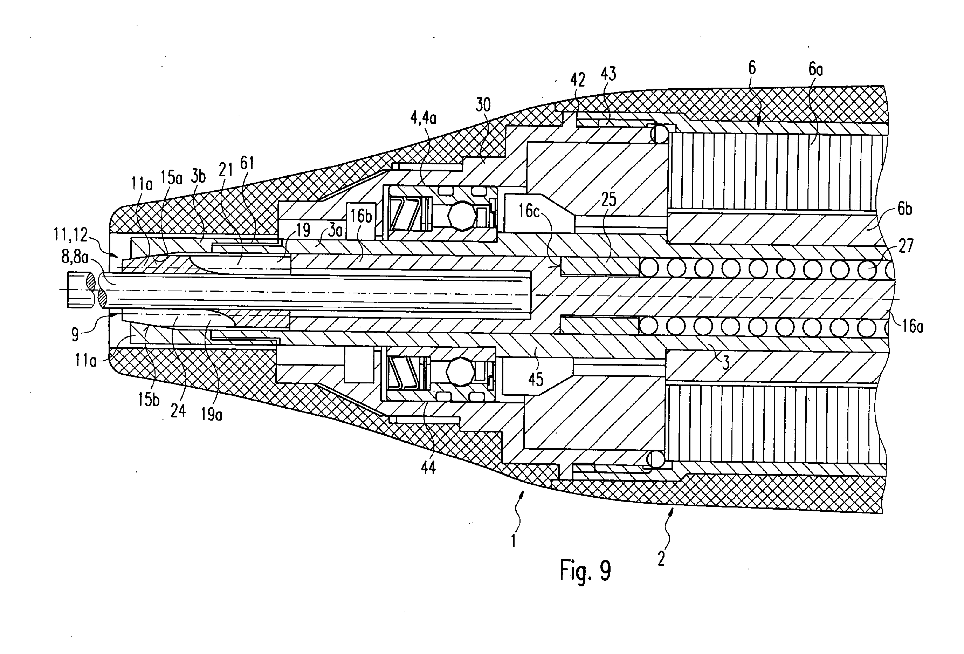

Beim Ausführungsbeispiel nach Fig. 9, bei dem gleiche oder vergleichbare Teile mit gleichen Bezugszeichen versehen sind, weist das Handstück 3 einen Werkzeughalter 12 auf, der durch eine Druck-Spannzange 11 gebildet ist, die dadurch betätigt wird, dass die Stange 16 für einen Spannvorgang nicht zurückgezogen sondern vorgeschoben wird und einen nach vorne gerichteten Druck auf die Spannzange ausübt. Auch bei dieser Ausgestaltung sind eine Gruppe, z. B. durch Spannsegmente 11a gebildete, Spannelemente mit zusammenwirkenden Konusflächen um das Steckloch 9 verteilt im Werkzeughalter 12 angeordnet und für einen Spannvorgang gegen das Steckloch 9 bewegbar, jedoch sind die Konusflächen, hier der Innenkonus 15a in der Antriebswelle 3 und die Außenkonusflächen 15b an den Spannsegmenten 11a zueinander passend nach vorne konvergent ausgebildet. Diese Konusteile sind vorzugsweise im vorderen Bereich der Spannzange 11 angeordnet.

Bei dieser Ausgestaltung ist eine Kupplung zwischen der Spannzange 11 und der Stange 16 nicht erforderlich, da eine Zugwirkung beim Spannen nicht stattfindet. Der Druck, der z.B. stumpf von hinten gegen die Spannzange 11 drückenden Stange 16, bei der es keiner Kupplung bedarf, reicht für die Spannfunktion aus. Die Spannzange 11 ist zwischen dem Innenkonus 15a und der Stange 16 auch formschlüssig unverlierbar gehalten. Auch bei dieser Ausgestaltung sind die Spannsegmente 11a an Federarmen 19a angeordnet, die durch auf dem Umfang verteilt angeordnete und nach vorne ausmündende Längsschlitze 24 gebildet sind. Dabei können auch hier nach hinten ausmündende Längsschlitze 21 angeordnet sein, die den Spannzangenkörper hinten in Segmente unterteilen.

Um die Montage der Spannzange 11 zu erleichtern, ist die in diesem Bereich hülsenförmig ausgebildete Antriebswelle 3 hinter dem Innenkonus 15a quer geteilt, und die Antriebswellenteile 3a, 3b sind durch eine lösbare Verbindung 61, insbesondere eine Schraubverbindung, miteinander verbunden.

Bei dieser abgewandelten Ausgestaltung ist die Stange 16 durch die Druckfeder 27 nach vorne in ihre Spannstellung vorgespannt. Die Spannzange 11 wird durch eine Zugbewegung der Stange 16 nach hinten gelöst, die durch den Lösemechanismus 13 erzeugbar ist, der sie entgegen der Kraft der Druckfeder 27 nach hinten bewegt.

Hierzu können z. B. die Nuten 33 eine entgegengesetzte Steigung haben, so dass das Druckglied 29 zum Lösen nach hinten bewegt wird und die Stange 16 nach hinten zieht, z. B. dadurch, dass die Stange 16 das Druckglied 29 hintergreift und dabei in einem Loch durchgreift und mit einem Kopfteil 16e hintergreift, so dass die Feder 27 die Spannelemente durch die Stange 16 nach vorne schiebt und spannt. Insoweit ist die Druck-Spannzange 11 in umgekehrter Weise wirksam wie die Zug-Spannzange 11 gemäß Fig. 1

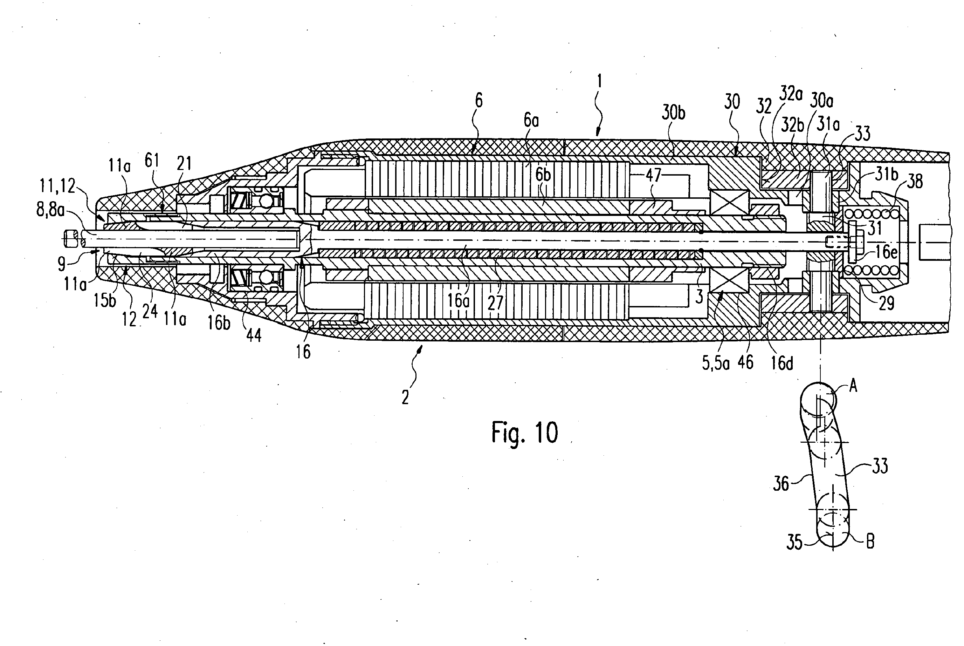

Die Spannwirkung dieses Werkzeughalters 12 lässt sich dadurch verbessern, gemäß dem Ausführungsbeispiel nach Fig. 10 dass zwei Gruppen von Spannelementen vorgesehen sind, die axial hintereinander und jeweils um das Steckloch 9 herum angeordnet und gegen das Steckloch 9 bewegbar sind. Dies lässt sich durch eine gemeinsame Druckbeaufschlagung der Gruppen erreichen, und zwar insbesondere dann in einfacher Weise, wenn in jeder Gruppe Spannsegmente angeordnet sind, von denen die Spannsegmente 11a der vorderen Gruppe nach vorne konvergente Außenkonusflächen 15b und die der hinteren Gruppe nach hinten konvergente Außenkonusflächen 15b aufweisen, wobei die Stange 16 an ihrem vorderen Ende ein insbesondere durch einen Innenkonus 15c gebildetes gemeinsames Druckglied aufweist, das mit den hinteren Außenkonusflächen 15b zusammenwirkt. Hierbei wirkt der Druck der Stange 16 gleichzeitig auf alle Spannelemente.

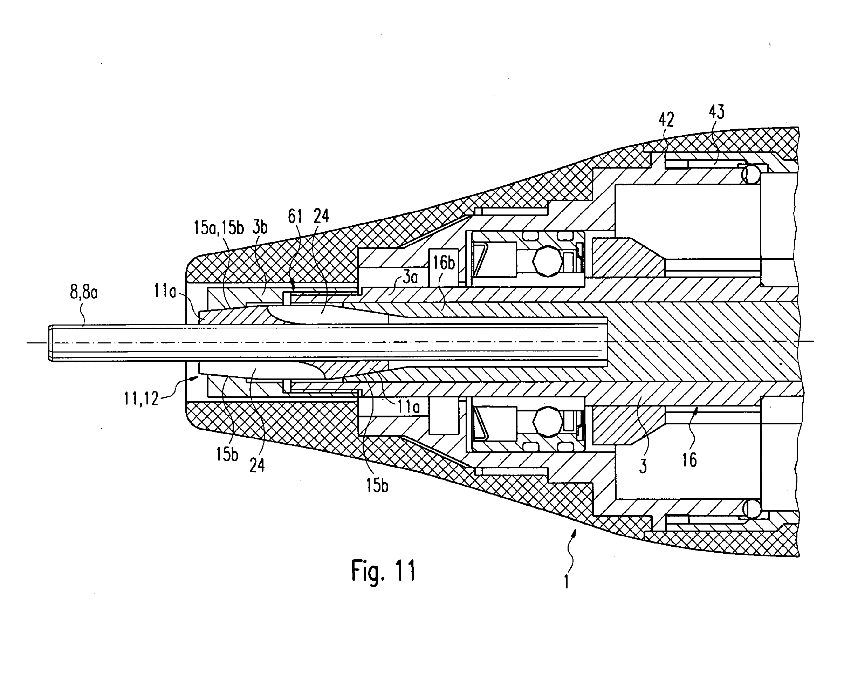

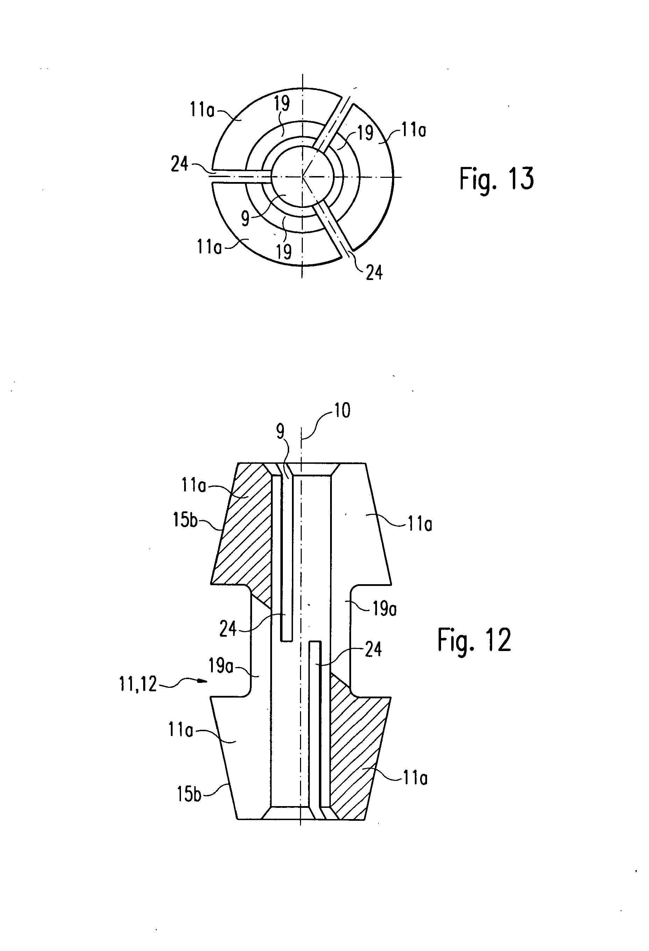

Wie insbesondere Fig. 11 und 12 zeigen, sind bei diesem Werkzeughalter 12 an beiden axialen Enden jeweils eine Gruppe von z. B. drei Spannsegmenten 11a jeweils an einem Federarm 19a gehalten und somit radial einwärts gegen die Federarmkraft bewegbar. Die Spannsegmente 11a und die Federarme 19a sind durch Längsschlitze 24 im Hülsenkörper gebildet, die jeweils endseitig ausmünden und deshalb den radialen Federweg gewährleisten. Die Längsschlitze 24 und die Spannsegmente 11a sind vorzugsweise in der Umfangsrichtung versetzt zueinander angeordnet. Hierdurch ist es möglich, die Schlitze 24 einander axial überlappend anzuordnen, insbesondere im mittleren Bereich des Hülsenkörpers und somit im Bereich zwischen den Gruppen der Spannsegmente 11a bzw. Spannelemente.

Die erfindungsgemäße Konstruktion ist wie bereits erwähnt nicht auf den Einsatz in medizinischen oder zahntechnischen Handstücken beschränkt. Stattdessen bringt die vorliegende Erfindung grundsätzlich gesehen immer dann Vorteile mit sich, wenn ein Werkzeughalter für ein rotierendes Werkzeug, der mit einer Antriebswelle gekoppelt ist, einfach montiert oder demontiert und die gesamte Anordnung möglichst kompakt gehalten werden soll. Dementsprechend wäre neben dem medizinischen Einsatz auch die Verwendung in anderen motorgetriebenen Systemen bzw. Motorelementen, beispielsweise in HF-Motorspindeln und dgl. denkbar.

## Patentansprüche

1. Motorelement, insbesondere medizinisches oder dentaltechnisches Handstück oder Motorspindel, das wenigstens in seinem vorderen Bereich einen länglichen Schaft aufweist, in dem eine hohle Antriebswelle (3) drehbar gelagert ist, in deren vorderem Endbereich eine Spannzange (11) für ein Werkzeug (8) angeordnet ist, die in ihrem hinteren Endbereich durch eine Kupplung (17) mit einer sich nach hinten erstreckenden Stange (16) verbunden ist, die durch die Kraft einer Feder (27) nach hinten oder nach vorne vorgespannt ist und durch einen Betätigungsmechanismus (13) gegen die Kraft der Feder (27) nach vorne oder nach hinten verschiebbar ist, wobei die Feder (27) nach hinten an der Stange (16) abgestützt ist und nach vorne gegen eine innere erste Schulter (52) der Antriebswelle (3) abgestützt ist,
**dadurch gekennzeichnet,**
**daß** am vorderen Endbereich der Feder (27) mindestens ein radial nach außen elastisch vorgespanntes Stützelement (25a) angeordnet ist, welches hinter die Schulter (52) ausfedert, wenn die Zugstange (16) mit der Feder von vorne in die Antriebswelle eingeschoben und das Stützelement (25a) in eine hinter der Schulter befindliche Position gelangt.

2. Motorelement nach Anspruch 1
**dadurch gekennzeichnet**,
das die Schulter (52) durch eine Ringnut gebildet ist.

3. Motorelement nach Anspruch 1 oder 2,
**dadurch gekennzeichnet,**
**dass** das Stützelement (52a) durch einen Federarm gebildet ist.

4. Motorelement nach einem der vorherigen Ansprüche,
**dadurch gekennzeichnet,**
**dass** mehrere auf dem Umfang verteilt angeordnete Stützelemente (25a) angeordnet sind.
